# EUROPÄISCHE PATENTSCHRIFT

(11) **EP 2 178 553 B1**
(45) Veröffentlichungstag und Bekanntmachung des Hinweises auf die Patenterteilung: **27.11.2013**
(21) Anmeldenummer: 08784323.1
(22) Anmeldetag: 14.07.2008
(51) Int. Cl.: A61K 38/17, A61P 25/02

(54) **VERWENDUNG EINES GRANULINS ODER EINER GRANULIN-ÄHNLICHEN VERBINDUNG ZUR THERAPIE ODER PROPHYLAXE VON CHRONISCHEN SCHMERZEN**
USE OF A GRANULIN OR A GRANULIN-LIKE COMPOUND IN THE THERAPY OR PROPHYLAXIS OF CHRONIC PAINS
UTILISATION D'UNE GRANULINE OU D'UN COMPOSÉ ANALOGUE À LA GRANULINE POUR LA THÉRAPIE OU LA PROPHYLAXIE DES DOULEURS CHRONIQUES

(30) Priorität: 16.07.2007 DE 102007033359
(43) Veröffentlichungstag der Anmeldung: 28.04.2010
(73) Patentinhaber: Johann Wolfgang Goethe-Universität Frankfurt am Main, 60325 Frankfurt am Main (DE)
(72) Erfinder: TEGEDER, Irmgard, 60598 Frankfurt (DE); GEISSLINGER, Gerd, 65812 Bad Soden (DE)
(74) Vertreter: Krauss, Jan
(86) Internationale Anmeldenummer: PCT/DE2008/001138
(87) Internationale Veröffentlichungsnummer: WO 2009/010045

(56) Entgegenhaltungen:
- EP-A- 1 164 144
- WO-A-93/15195
- WO-A-2004/078782
- WO-A-2007/000924
- WO-A-2008/019187
- WANG W ET AL: "PC cell-derived growth factor (Granulin precursor) expression and action in human multiple myeloma" CLINICAL CANCER RESEARCH, THE AMERICAN ASSOCIATION FOR CANCER RESEARCH, US, Bd. 9, 1. Juni 2003 (2003-06-01), Seiten 2221-2228, XP002977715 ISSN: 1078-0432

## Beschreibung

Die Erfindung betrifft die Verwendung eines Granulins oder einer Granulin-ähnlichen Verbindung zur Herstellung einer pharmazeutischen Zusammensetzung zur Therapie oder Prophylaxe von chronischen Schmerzen, insbesondere zur Herstellung von pharmazeutischen Zubereitungen zur Therapie oder Prophylaxe von chronischen Schmerzen. Weiterhin betrifft die Erfindung eine entsprechende pharmazeutische Zusammensetzung.

Bei Patienten, die eine Nervenschädigung, sei sie traumatisch, entzündlich, infektiös, tumorbedingt, ischämisch, degenerativ, metabolisch oder toxisch, erlitten haben, kommt es in Folge dieser Nervenschädigung häufig zu sekundären neuronalen Schädigungen, wie Zelltod und chronischen Schmerzen, insbesondere zu neuropathischen Schmerzen.

Neuropatische Schmerzen können durch vielfältige Schädigungen peripherer und zentraler Nervenzellen entstehen. Häufig sind unter anderem Schmerzen bei diabetischer oder toxischer (etwa in Folge einer Chemotherapeutikatherapie) Polyneuropathie, Phantomschmerzen nach Amputation, postzosterische Neuralgie, Trigeminus Neuralgie, Kompressionssyndrome, wie das Karpaltunnelsyndrom und Ischialgie bei Bandscheibenprolaps, Schmerzen bei Multipler Sklerose und postischämische Neuralgie. Die nichtinvasiven medikamentösen Möglichkeiten, die aus dem Stand der Technik bekannt sind, beschränken sich auf den Einsatz verschiedener Antiepileptika (z. B. Gabapentin, Pregabalin), Antidepressiva (z. B. Amitrytilin) und Opioide, die jedoch bei nur etwa 30 % der Patienten einen zufriedenstellenden analgetischen Effekt bei gleichzeitig tolerierbaren Nebenwirkungen erzielen können und die neuronale Schädigung nicht beeinflussen.

Die Mechanismen, die zur Entstehung chronischer, insbesondere neuropatischer Schmerzen beitragen, sind vielfältig. Experimentelle Untersuchungen der letzten Jahre zeigen, dass der transsynaptische Untergang inhibitorischer Neurone und die Aktivierung von Gliazellen essenziell dazu beitragen.

Demgemäß lag der vorliegenden Erfindung die Aufgabe zugrunde, ein Verfahren bzw. ein Mittel bereitzustellen, dass zur Therapie oder Prophylaxe einer Nervenschädigung eingesetzt werden kann, um insbesondere die Regeneration bei partieller peripherer Nervenläsion zu begünstigen und den sekundären Zelltod zu verhindern.

Der Kern der vorliegenden Erfindung besteht darin, durch therapeutischen Einsatz einer neuroprotektiven Verbindung den Verlust inhibitorischer Neurone zu reduzieren und die Gliavermittelte Entzündungsreaktion zu hemmen, um so eine fortschreitende Schädigung durch dauernde Übererregung zu verhindern. Dies reduziert chronische, insbesondere neuropatische Schmerzen und begünstigt Regenerationsprozesse.

Das der Erfindung zu Grunde liegende Problem wird durch die Verwendung eines Granulins oder einer Granulin-ähnlichen Verbindung zur Herstellung einer pharmazeutischen Zusammensetzung zur Therapie und/oder Prophylaxe von chronischen Schmerzen gelöst. Dabei umfasst der Begriff "chronischer Schmerz" u. a. neuropathische Schmerzen sowie inflammatorische und tumorbedingte Schmerzen.

Die Verwendung von Granulin oder einer Granulin-ähnlichen Verbindung zur Therapie oder Prävention chronischer, insbesondere neuropatischer Schmerzen bei einer akuten oder chronischen Nervenschädigung stellt ein neues therapeutisches Prinzip dar, dass im Gegensatz zu den verfügbaren medikamentösen Möglichkeiten (Antiepileptika, Antidepressiva, Opioide und Cannabioide) die pathophysiologisch zugrundeliegenden Mechanismen positiv beeinflusst.

Erfindungsgemäß gelingt es, den Untergang inhibitorischer Interneurone zu verhindern. Damit wird die mangelnde Hemmung exzitatorischer Impulse und somit die Überaktivität schmerzleitender Bahnen verhindert. Durch die sich daraus ergebene Begrenzung der Umbauprozesse und Fehlverschaltungen, die in Folge der Apoptose inhibitorischer Neurone auftreten, wird die Chronifizierung neuropatischer Schmerzen reduziert. Im Gegensatz dazu führen die bisher zur Verfügung stehenden Medikamente zu einer generellen Dämpfung neuronaler Aktivität und beeinflussen die pathophysiologischen Mechanismen bei neuropatischen Schmerzen in Folge peripherer oder zentralen Nervenschädigungen nicht.

In der Erfindung ist das Granulin oder die Granulin-ähnliche Verbindung mindestens ein Molekül, das ausgewählt ist aus einer Gruppe enthaltend: Progranulin, Granulin 1, Granulin 2, Granulin 3, Granulin 4, Granulin 5, Granulin 6, Granulin 7, Paragranulin, Progranulinsignalmolekül, zu den vorgenannten Moleküle gleichwirkenden Fragmenten dieser Moleküle.

Auch die Verwendung von Kombinationen von verschiedenen Molekülen aus der oben genannten Gruppe wird von der Erfindung umfasst.

Grundsätzlich bezieht sich die Erfindung sowohl auf Proteine bzw. Peptide und Nukleinsäuren, wie DNA, cDNA oder RNA, sowie deren Derivate.

Demgemäß kann in einer Ausgestaltung der Erfindung das Granulin oder die Granulin-ähnliche Verbindung eine für ein der genannten Moleküle kodierende Nukleinsäure sein. Insbesondere kann die Nukleinsäure eine Nukleinsäure gemäß einer der SEQ. ID. NOs. 1 bis 10 oder eine zu einer dieser Sequenzen zu mindestens 50 %, bevorzugt zu 60 %, 70 % oder 80 %, insbesondere bevorzugt zu 85 % oder 90 %, am meisten bevorzugt zu 95 % oder 98% identische Nukleinsäure sein. Dabei ist das von der Nukleinsäure kodierte Peptid oder Protein vorteilhafter Weise gleichwirkend zu einem Granulin oder einer Granulin-ähnlichen Verbindung gemäß SEQ. ID. NO. 11 bis 20.

Unter einem Fragment einer Nukeinsäure wird eine beliebige in einer Nukleinsäure kodierend Granulin oder einer Granulin-ähnlichen Verbindung vorkommende Abfolge von mindestens 50, bevorzugt von mindestens 150, besonders bevorzugt von mindestens 180 zusammenhängenden Nukleotiden aus der Sequenz von Granulin oder einer Granulin-ähnlichen Verbindung verstanden.

Die für ein gleichwirkendes Fragment kodierende Nukleinsäure ist bezüglich der für dieses Fragment kodierenden Nukleinsäure aus SEQ. ID. NO. 11 bis 20 zu mindestens 50 % identisch, bevorzugt zu 60 %, 70 % oder 80 %, insbesondere bevorzugt zu 85 % oder 90 %, am meisten bevorzugt zu 95 % oder 98 % identisch.

Als "gleichwirkend" wird ein Fragment verstanden, dass auf Neuronen und Gliazellen die gleiche qualitative Wirkung zeigt wie ein Granulin oder eine Granulin-ähnlichen Verbindung gemäß SEQ. ID. NOs. 11 bis 20, wobei die quantitative Wirkung abweichend sein kann.

Alternativ dazu kann in einer anderen Ausgestaltung der Erfindung das Granulin oder die Granulin-ähnliche Verbindung ein Protein oder ein Peptid sein.

In einer besonders bevorzugten Ausführungsform ist das Granulin oder die Granulin-ähnliche Verbindung ein Protein oder ein Peptid gemäß einer der SEQ. ID. NOs. 11 bis 20 oder eine zu einer dieser Sequenzen zu mindestens 50 %, bevorzugt zu 60 %, 70 % oder 80 %, insbesondere bevorzugt zu 85 % oder 90 %, am meisten bevorzugt zu 95 % oder 98 % identisches Protein oder Peptid.

Unter einem Fragment von Proteinen oder Peptiden wird eine beliebige in einem Granulin oder einer Granulin-ähnlichen Verbindung vorkommende Abfolge von mindestens 5, bevorzugt von mindestens 10 oder mindestens 20, besonders bevorzugt von mindestens 30, 40, am meisten bevorzugt von mindestens 50 zusammenhängenden Aminosäuren aus der Sequenz von Granulin oder einer Granulin-ähnlichen Verbindung verstanden.

Die oben beschriebene Verwendung eignet sich zur Herstellung von pharmazeutischen Zubereitungen zur Therapie oder Prophylaxe von chronischen, insbesondere neuropathischen Schmerzen bei Säugetieren, insbesondere beim Menschen, aber auch bei Haus- und Nutztieren. Die vorliegende Erfindung wird zwar in Bezug auf humane Nukleinsäure- und Protein-/Peptidsequenzen beschrieben. Der Fachmann kann aber auf der Grundlage der humanen Sequenzen unter Verwendung bekannter Methoden auch die Sequenzen anderer Spezies ermitteln, sofern sie nicht in den öffentlich zugänglichen Datenbanken enthalten sind.

Die oben beschriebene Verwendung eignet sich ebenfalls zur Herstellung von pharmazeutischen Zubereitungen zur Reduktion neuropathischer Schmerzen.

Das der Erfindung zu Grunde liegende Problem wird ebenfalls durch eine pharmazeutische Zusammensetzung gelöst, die ein Granulin oder ein gleichwirkendes Granulinfragment.

Dabei ist das Granulin oder die Granulin-ähnliche Verbindung mindestens ein Molekül, das ausgewählt ist aus einer Gruppe enthaltend Progranulin, Granulin 1, Granulin 2, Granulin 3, Granulin 4, Granulin 5, Granulin 6, Granulin 7, Paragranulin, Progranulinsignalmolekül, zu den vorgenannten Molekülen gleichwirkende Fragmente dieser Moleküle.

Analog zu der obigen Beschreibung kann das Granulin oder die Granulin-ähnliche Verbindung der pharmazeutischen Zusammensetzung eine für eines der genannten Moleküle kodierende Nukleinsäure sein, insbesondere eine Nukleinsäure gemäß einer der SEQ. ID. NOs. 1 bis 10 oder eine zu einer dieser Sequenzen zu mindestens 50 %, bevorzugt zu 60 %, 70 % oder 80 %, insbesondere bevorzugt zu 85 % oder 90 %, am meisten bevorzugt zu 95 % oder 98% identische Nukleinsäure sein. Dabei ist das kodierte Peptid oder Protein gleichwirkend im Sinne der obigen Definition.

Die genannten Nukleinsäuren können einem Patienten durch Transfer eines die genannten Sequenzen aufweisenden Konstruktes, wie beispielsweise Viren oder Plasmide in mindestens eine Zelle verabreicht werden. Die Transfektion der Konstrukte kann mittels bekannter Methoden wie Lipofektion, Ballistischen Transfer ("gene gun") etc. erfolgen.

In einer alternativen Ausgestaltung ist das Granulin oder die Granulin-ähnliche Verbindung der pharmazeutischen Zusammensetzung ein Protein oder ein Peptid. Dabei ist es von Vorteil, wenn das Granulin oder die Granulin-ähnliche Verbindung ein Protein oder ein Peptid gemäß einer der SEQ. ID. NOs. 11 bis 20 oder eine zu einer dieser Sequenzen zu mindestens 50 %, bevorzugt zu 60 %, 70 % oder 80 %, insbesondere bevorzugt zu 85 % oder 90 %, am meisten bevorzugt zu 95 % oder 98% identisches Protein oder Peptid ist.

Die beschriebene pharmazeutische Zusammensetzung kann bevorzugt zur Herstellung von pharmazeutischen Zubereitungen zur Therapie oder Prophylaxe von neuropathischen Schmerzen bei Säugetieren, insbesondere bei Haus- und Nutztieren und beim Menschen verwendet werden. Obwohl die hier angegebenen Sequenzen alle vom Menschen stammen, kann der Fachmann anhand dieser angegebenen Sequenzen die entsprechenden Moleküle in anderen Organismen identifizieren und isolieren.

Die pharmazeutische Zusammensetzung kann erfindungsgemäß auch zur Herstellung von pharmazeutischen Zubereitungen zur Reduktion neuropathischer Schmerzen.

Die pharmazeutische Zusammensetzung können in Form von Tabletten, Dragees, Pillen, Granulaten, Aerosolen, Infusionslösungen, Emulsionen, Suspensionen oder Lösungen vorliegen.

Die erfindungsgemäße Verwendung der Mittel bzw. der pharmazeutischen Zusammensetzung kann durch geeignete bekannte Formulierungen geschehen.

Die erfindungsgemäße Verwendung der Mittel kann in bekannter Weise in die üblichen Formulierungen überführt werden, wie Tabletten, Dragees, Pillen, Granulate, Aerosole, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Mittelkonzentration in Bezug auf das Granulin oder die Granulin-ähnlichen Verbindungen jeweils in einer Konzentration von etwa 0,1 Gew.% bis 95 Gew.-%, bevorzugt von etwa 0,5 Gew.-% bis 90 Gew.-% der Gesamtmischung vorhanden sein, d. h. in Mengen, die ausreichend sind, um die erforderlichen Wirkstoffkonzentrationen im Zielgewebe zu erreichen.

Die Formulierungen werden beispielsweise hergestellt durch Verstrecken der Mittel mit Lösungsmitteln und/oder Trägerstoffen, gegebenenfalls unter Verwendung von Emulgiermitteln und/oder Dispergiermitteln, wobei z. B. im Fall der Benutzung von Wasser als Verdünnungsmittel gegebenenfalls organische Lösungsmittel als Hilfsstoffe verwendet werden können.

Als Hilfsstoffe erwähnt seien z. B. Wasser, nicht-toxische Lösungsmittel wie Parafin (z.B. Erdölfraktionen), pflanzliche Öle (z. B. Erdnuß-, Sesamöl), Alkohole (z. B. Ethylalkohol, Glycerin), Trägerstoffe, wie natürliche Gesteinsmehle (z. B. Kaoline, Tonerde, Talkum, Kreide), synthetische Gesteinsmehle (z. B. hochdisperse Kieselsäure, Silikate), Zucker (z. B. Rohr-, Milch- und Traubenzucker), Emulgiermittel (z. B. Polyoxyethylen-Fettsäure-Ester, Polyoxyethylen-Fettalkohol-Ether), Dispergiermittel (z. B. Lignin, Sulfitablaugen, Methylcellulose, Stärke und Polyvinylpyrolidon) und Gleitmittel (z. B. Magnesiumstearat, Talkum, Stearinsäure und Natriumsulfat).

Die Applikation erfolgt in üblicher Weise, bevorzugt oral oder parenteral, insbesondere perlingual oder intravenös. Im Falle der oralen Anwendung der erfindungsgemäßen Medikamente können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine, und dergleichen enthalten. Weiterhin können Gleitmittel, wie Magnesiumstearat, Natriumlaurylsulfat und Talkum zum Tablettieren mitverwendet werden. Im Falle wässriger Suspensionen können die Wirkstoffe außer den oben genannten Hilfsstoffen mit verschiedenen Geschmacksaufbesserern oder Farbstoffen versetzt werden. Für den Fall der parenteralen Anwendung können Lösungen der Wirkstoffe unter Verwendung geeigneter flüssiger Trägermaterialien eingesetzt werden.

Die Erfindung betrifft auch die Verwendung eines Granulins oder einer Granulin-ähnlichen Verbindung wie hier beschrieben zur Therapie oder Prophylaxe von neuropathischen Schmerzen, bzw. ein Granulin oder einer Granulin-ähnliche Verbindung wie hier beschrieben und seine Verwendung zur Therapie oder Prophylaxe von neuropathischen Schmerzen.

Die Erfindung wird anhand von Beispielen näher erläutert, ohne auf diese Beispiele beschränkt zu sein. Die Ergebnisse der in den Beispielen beschriebenen Versuche sind in den Figuren dargestellt.

### Figuren

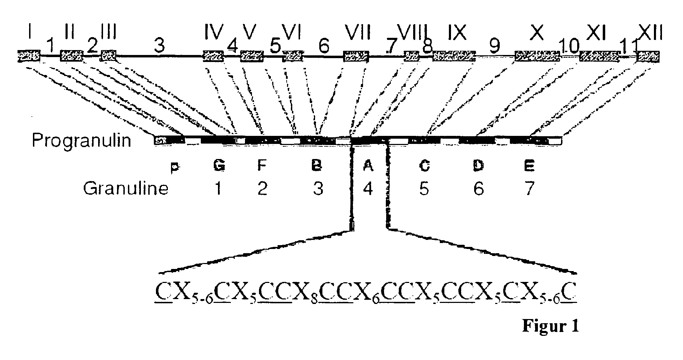

- Figur 1:: Progranulin ist ein cysteinreiches Protein, das durch bisher unbekannte Proteasen in sieben verschiedene Granuline gespalten wird (Abb.1), die sezerniert werden, wie das Ausgangsprodukt Wachstumsfaktor- und Cytokin-ähnliche Effekte haben, die Wundheilung fordern, Tumorwachstum und neuronale Entwicklung regulieren. Extra- und intrazelluläre "Rezeptoren" sind bislang nicht bekannt. Beim Menschen bedingt eine "loss-of-function" Mutation des Progranulin-Gens eine frontotemporale Demenz.
Intron-Exon-Struktur des humanen Progranulin-Gens Grn, Exons sind als Boxen dargestellt. Progranulin-Protein wird durch Proteasen in 7 verschiedene Granuline (schwarze Boxen) und ein N-terminales "signaling peptide" gespalten. Alle Granuline haben eine dem Granulin A ähnliche Struktur (Cx-Cx-CCx-CCx-CCx-CCx-Cx-C). C = Cystein, X = variable Anzahl anderer Aminosäuren.

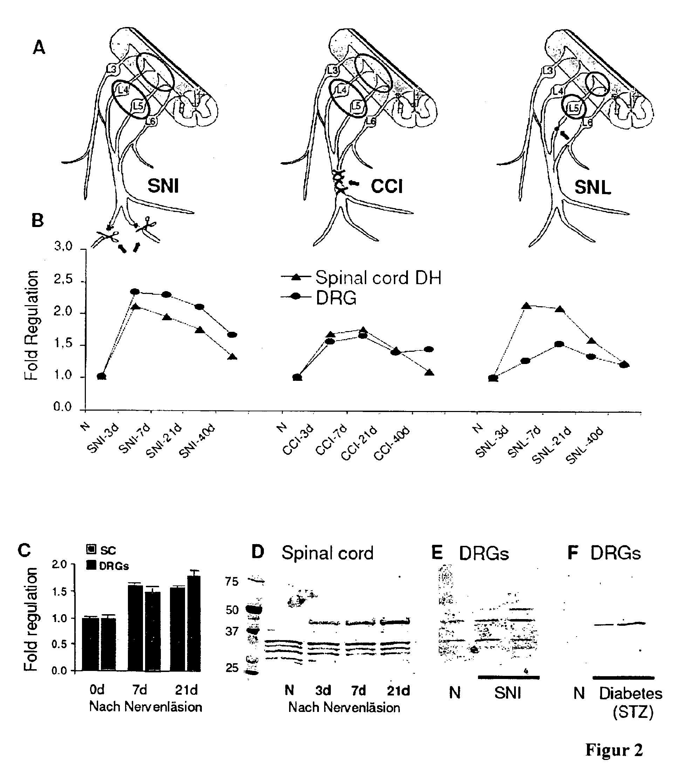

- Figur 2:: Regulation der Progranulin- Expression in Rückenmark und DRGs nach peripherer Läsion der N. ischiadicus.
**A** Neuropathische Modelle, Spared Nerve Injury (SNI, Decosterd & Woolf), Chronic Constriction Injury (CCI, Bennett), Spinal Nerve Ligation (SNL, Chung).
**B** Microarray Analyse der Progranulin mRNA in Dorsalhorn und DRGs in den oben gezeigten Modellen.
**C** Quantitative RT-PCR der Progranulin mRNA im SNI Modell.
**D** Western Blot Analyse der Progranulin Protein-Expression im Rückenmark im SNI Modell.
**E** Progranulin-Protein in DRGs nach SNI.
**F** Progranulin-Proteinexpression in DRGs im STZ induzierten Diabetes Polyneuropathie-Modell, 3 Monate nach Diabetes Induktion.

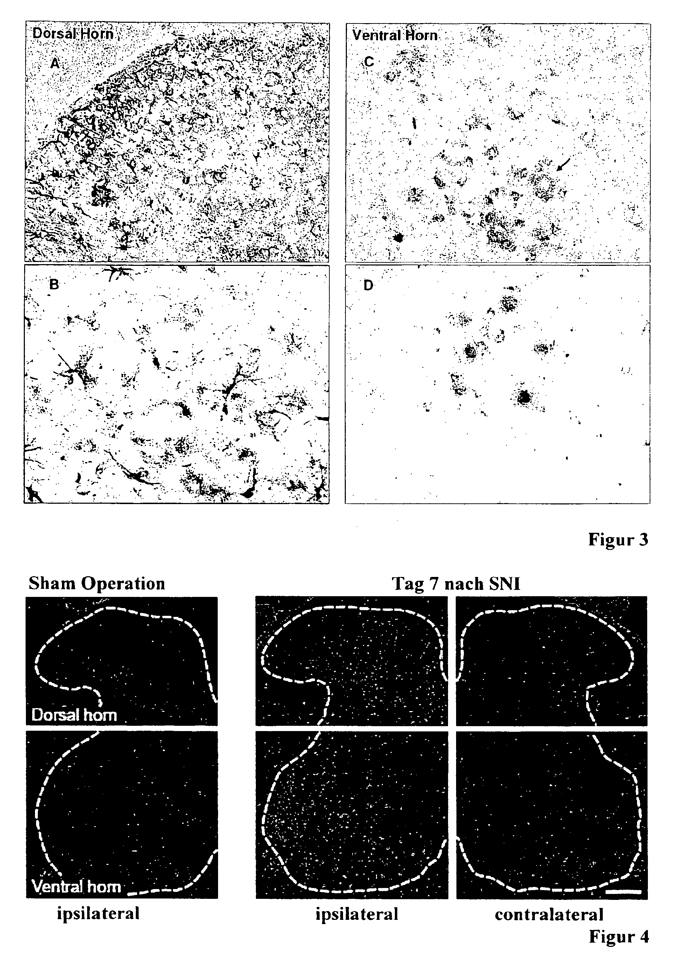

- Figur 3:: Progranulin in situ Hybridisierung und post-in situ Immunfluoreszenz für glial fibrillary acid protein, GFAP und den Transkriptionsfaktor ATF-3.
**A** *Grn* in situ (schwarz) und GFAP Immunfuoreszenz (rot) zur Darstellung der Astrozyten nach SNI im Dorsalhorn des Rückenmarks.
**B** Höhere Vergrößerung von A. Progranulin wird nicht in Astrozyten exprimiert.
**C** *Grn* in situ (schwarz) in Mikroglia (kleine Zellen) und Motoneuronen (Pfeil) im Vorderhorn des Rückenmarks nach SNI.
**D** Vorderhorn wie in C mit Darstellung geschädigter Motoneuronen durch ATF-3 Immunfluoreszenz (rot). ATF-3 ist ein Marker für Neuronen mit geschädigtem Axon.
- Figur 4:: Übersicht der Mikrogliareaktion im Rückenmark nach SNI. Darstellung der Mikroglia durch Immunfluoreszenz für den Rezeptor des Komplementfaktors 3 (CD11b).

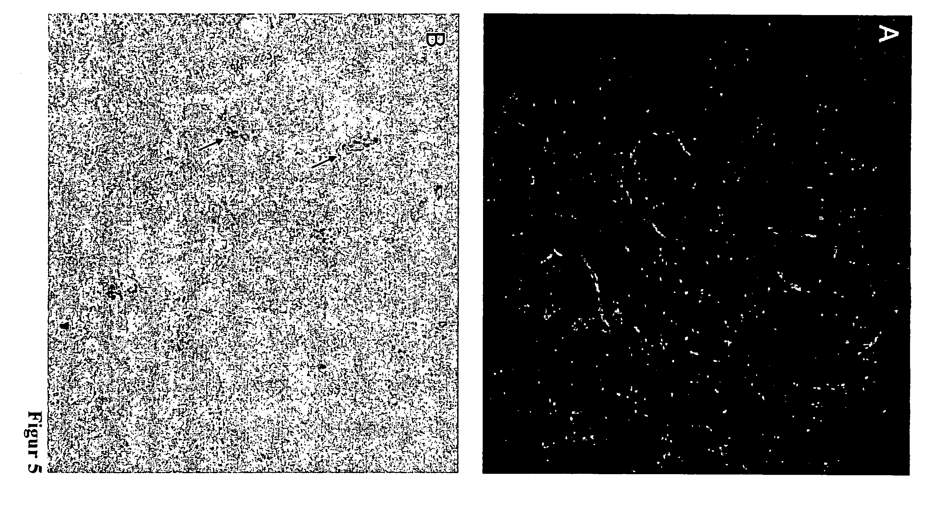

- Figur 5:: Mikrogliareaktion im Vorderhorn nach SNI
**A** CD11b Immunfuoreszenz zur Darstellung aktivierter Mikrogliazellen im Ventralhom des Rückenmarks nach SNI.
**B** Progranulin mRNA (in situ Hybridisierung, grau) in aktivierten Mirkogliazellen (Pfeil) neben Motoneuronen (rot, Peripherin-Immunfluoreszenz).

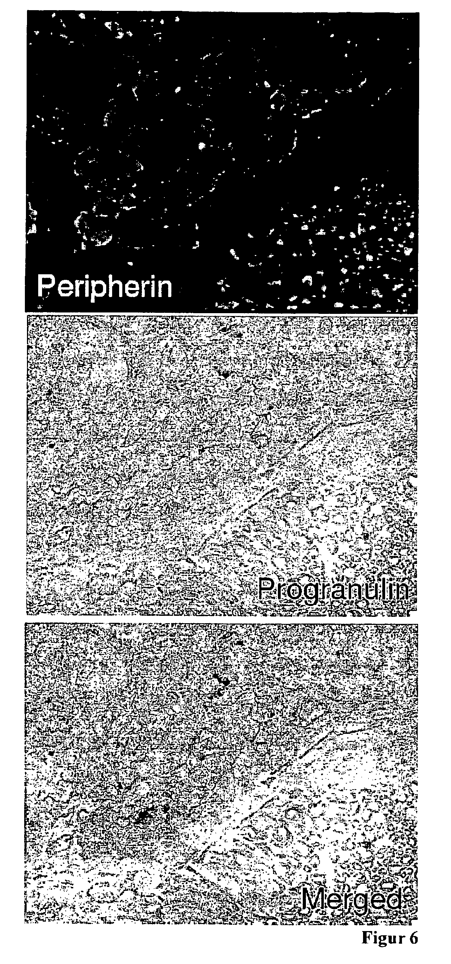

- Figur 6:: Neuronen der Dorsalganglien (Peripherin IR rot) mit Expression der Progranulin mRNA (in situ Hybridisierung, grau) nach SNI.

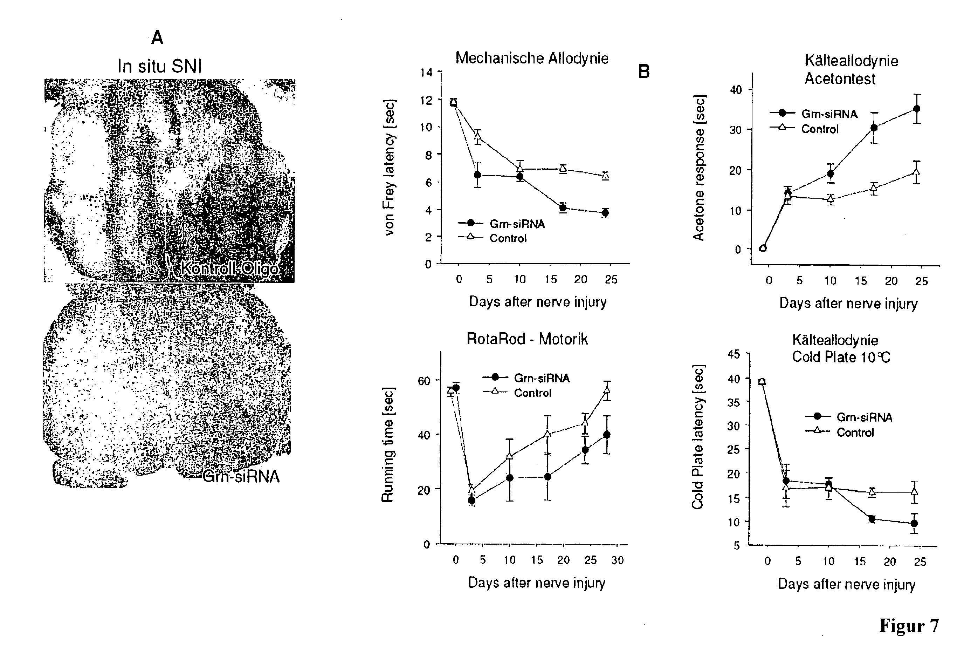

- Figur 7:: Progranulin mRNA Expression und Verhalten von Mäusen bei kontinuierlicher spinaler Infusion von Progranulin siRNA nach SNI.
**A** Grn mRNA nach SNI im Rückenmark bei Behandlung mit Grn siRNA und Kontroll-Oligonukleotiden. Die Behandlung erfolgt mit Hilfe einer subkutan implantierten osmotischen Alzet Pumpe über einen spinalen Katheter, der in Höhe von L3 endet. Die Infusion beginnt unmittelbar nach SNI und endet 4 Wochen nach SNI.
**B.** Verhalten der Mäuse (C57Black6) nach SNI bei Behandlung mit Grn siRNA oder Kontrolloligonukleotiden in verschiedenen nozizeptiven Tests und im RotaRod Test, der die motorische Leistung erfasst. Mechanische Allodynie wurde durch Messen der "Withdrawal"-Latenz mit einem dynamischen von Frey Ästhesiometer gemessen, je kürzer die Latenz, desto stärkere Allodynie (oben links). Kälteallodynie wurde im Acetontest (oben rechts) und mit einer Cold Plate bei 10°C (unten rechts) erfasst. Im Acetontest wird die Dauer der Reaktion (Lecken, Schütteln, Anheben) der Pfote nach Applikation eines Tropfen Acetons auf die Fußsohle gemessen, je länger die Reaktion, desto stärker die Allodynie. Mit der Cold Plate wird die Reaktionslatenz nach Kälteexposition gemessen, je kürzer desto stärkere Allodynie. Im RotaRod Test wird gemessen, wie lange die Maus auf einer sich drehenden Walze läuft ohne herunterzufallen. Gemessen wird motorische Koordination und Ermüdung sowie Fähigkeit zur Kompensation nach Nervenläsion. Die Verhaltensversuche zeigen Zunahme der Allodynie und beeinträchtigte motorische Erholung bei Behandlung mit Grn siRNA. Die Behandlung von Mäusen durch spinale Infusion von Progranulin siRNA blockiert die nach SNI auftretende Hochregulation des Progranulin und führt zu einer Zunahme des Schmerzähnlichen Verhaltens bei Mäusen. Zudem ist die Erholung der motorischen Funktion im RotaRod Test etwas langsamer als bei Kontrolltieren, die mit KontrollOligonukleotiden behandelt wurden.

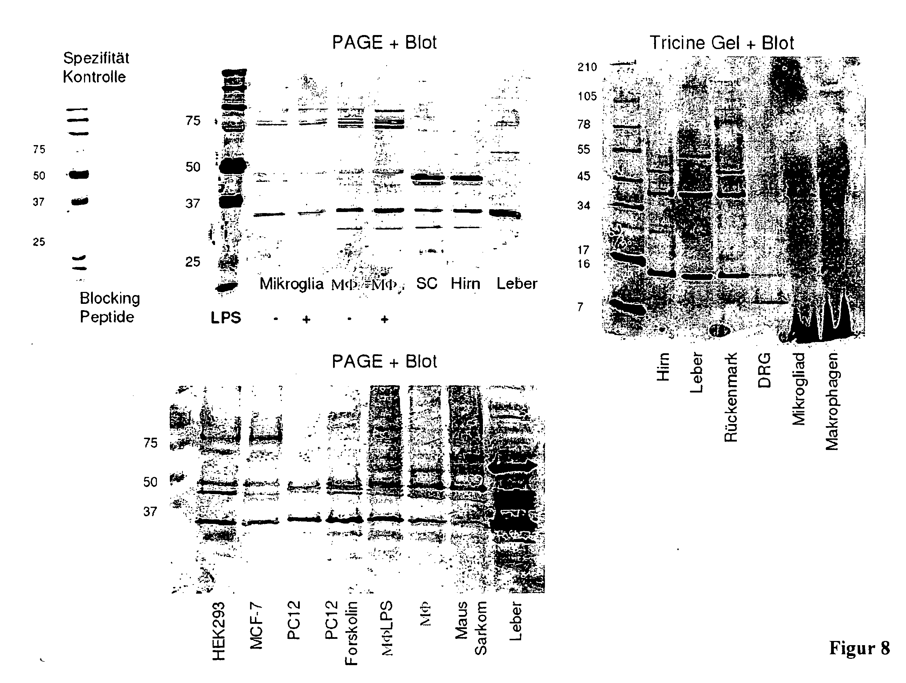

- Figur 8:: Progranulin/Granulin Protein Expression in Zellen und Mausgeweben. Western Blot mit N19- Antikörper (SantaCruz Biotechnology) gerichtet gegen Progranulin, Granulin 1, 2 und 7. Molekulargewicht des glycosyliertes Progranulin 88 kDa, der einzelnen Granuline 6 kDa. Tricine Gele wurden zur Auftrennung der kleinen Peptide verwendet. Abkürzungen: MF Makrophagen, LPS Lipopolysaccharid, SC Spinal Cord, DRG Dorsalganglien, HEK293 Human embryonic kidney cells, MCF-7 breast cancer cells, PC12 Phäochromocytoma cells (Neuronen-ähnlich), PAGE Polyacrylamid-Gel-Elektrophorese.

### Beispiele

Im Folgenden wird die Erfindung mit Bezug auf die in den Figuren dargestellten Ergebnisse mittels der hier beschriebenen Experimente erläutert.

### Methoden Progranulin

### Neuropathiemodelle und RNAi Behandlung

Es wurden adulte C57B16 Mäuse verwendet. Im Spared Nerve Injury (SNI) Modell werden zwei Äste des Nervus (N.) ischiadicus, nämlich der N. peronaeus und N. tibialis ligiert und distal durchtrennt. Der N. suralis bleibt intakt. Silencer RNA (stealth RNAi, Invitrogen) für Acrogranin wurde mit Hilfe einer osmotischen Pumpe (Alzet osmotic pump 2004; 0,25 µl/h) kontinuierlich über einen spinalen Katheter (PTFE Sub-Lite Wall Tubing; OD/ID 0,15 mm/0,05 mm) intrathekal infundiert. Die Katheterspitze wurde in Höhe des Lumbalmarkes platziert, die osmotische Pumpe im Subkutangewebe fixiert. Die Infusion startete direkt nach SNI Operation und dauerte 4 Wochen. Eine Mischung aus drei verschiedenen Acrogranin RNAi (je 1 nmol pro Maus für den Zeitraum von 4 Wochen) bzw. Kontroll RNAi wurde verwendet. Zusätzlich wurde eine FITC gelabelte Dummy-RNAi (20 nmol) zur späteren immunhistologischen Lokalisation infundiert. Die Infusionslösung bestand aus Ringer Lösung mit 1:40 verdünntem Lipofectamin (195 µl Ringer + 5 µl Lipofecatmin pro Pumpe).

### Nozizeptives Verhalten

Die Tiere wurden für 3 x 30 min an die Testkäfige gewöhnt. Die Messung des Verhaltens wurde ohne Kenntnis der Behandlung durchgeführt. Die Reaktionslatenz auf mechanische Stimulation der Hinterpfote wurde mit Hilfe eines dynamischen von Frey Gerätes (Dynamic Aesthesiometer, UgoBasile, Italien) gemessen. Mit Hilfe einer "Cold Plate" (4 °C) wurde die Kälteschmerzempfindlichkeit durch Zählen der Abwehrreaktionen (Lecken, Springen, Bein anheben) über einen Zeitraum von 90 sec gemessen. Mit einer 52 °C "Hot Plate" wurde die Hitzeschmerzempfindlichkeit durch Messen der Latenz bis zur ersten Reaktion festgestellt. Die cut-off Latenz betrug 30 sec, um Gewebeschäden zu vermeiden. Im RotaRod Test wurde die motorische Koordination bzw. motorische Erholung nach Nervenläsion gemessen (90 sec cut-off).

### Microarrays

Die Gesamt-RNA wurde aus homogenisiertem Rückenmark bzw. DRG Gewebe mittels Qiagen RNA-Extraktionskit extrahiert und in DNA umgeschrieben. cDNA Fragmente wurden mittels PCR amplifiziert und in den pCR4 Vektor ligiert (TA Cloning Kit, Invitrogen). Biotinylierte cRNA wurde durch in vitro Transkription produziert und für die Hybridisierung der Affymetrix RGU34A Chips eingesetzt.

### Quantitative RT-PCR

Die Gesamt-RNA wurde aus homogenisiertem Rückenmark bzw. DRG Gewebe mittels Qiagen RNA-Extraktionskit extrahiert und in DNA umgeschrieben. Die quantitative real-time rt-PCR wurde mit Hilfe des Sybr-Green Detektionssystems entsprechend der Anleitung des Herstellers (Applied Biosystems) durchgeführt. Primer-Sets wurden mit Primer Express ausgewählt. Die Amplifikation spezifischer PCR-Produkte wurde durch Agarose Gelelektrophorese überprüft.

### Western Blot

Rückenmark bzw. DRG Gewebe wurde in PhosphoSafe Extraktionspuffer (Novagen) homogenisiert, das Homogenat zentrifugiert (40.000 g, 20 min) und Proteine im Überstand durch Polyacrylamid bzw. Tricin-Gel Elektrophorese aufgetrennt. Die Proteine wurden mittels Nass-Blot auf Nitrozellulose-membranen übertragen, geblockt in PBST-5 % Milch (0,05 % Tween 20 in 1 mol/l PBS mit 5 % fettarmen Milchpulver), über Nacht mit Antikörpern gegen Acrogranin (1:1000 in PBST) inkubiert, gewaschen, mit Sekundärantikörpern 2 Stunden (h) bei Raumtemperatur inkubiert und mit Hilfe des Odyssee Western Blot Scanners ausgewertet.

### in situ Hybridisierung

Mäuse wurden durch CO₂ und Entbluten getötet, L4/5 DRGs und Rückenmark schnell entnommen, in OCT eingebettet, auf Trocheneis gefroren, und mit einem Kryotom 14 µm Schnitte geschnitten. Schnitte wurden in 4 % Paraformaldehyd postfixiert und acetylatiert. DIG-markierte Riboproben wurden durch in vitro Transkription von cDNA Fragmenten und Einbau Digoxigenin-markierter dUTP generiert (Dig-labeling kit, Roche). Die Schnitte wurden für 2 h bei RT in Prähybridisierungslösung (5xSSC, 50 % Formamid, 2xDenhardt's, 500 µg/ml herring sperm DNA, 250 µg/ml yeast tRNA) prähybridisiert und anschließend mit 250 ng/ml Riboprobe (antisense und sense) in Prähybridisierungslösung für 16 h bei 72 °C im Ofen hybridisiert. Die Schnitte wurden in 0.2 x SSC bei 68 °C für 2 h gewaschen, mit anti-Dig-AP-Antikörper (1:1000 in 1 % Blocking Reagent (Roche) in 0,1 mol/l Maleinsäurepuffer) bei 4 °C über Nacht inkubiert und mit NBT/BCIP/Levamisol (Boehringer Mannheim) entwickelt. Die Schnitte wurden in Glycerol/Gelatin eingebettet oder durch *post in situ* Immunfluoreszenz mit entsprechenden Antikörpern weiter entwickelt.

### Immunfluoreszenz

Zur Fixation wurden die Tiere mit 1 x PBS und anschließend 4 % Paraformaldehyd intrakardial perfundiert. Das Gewebe wurde präpariert, in 4 % PFA für 2h nachfixiert, über Nacht in 20 % Sucrose in 1 x PBS gegen Kryoartefakte geschützt, in OCT eingebettet und 12 bzw. 14 µm Schnitte mit einem Kryotom geschnitten. Die Schnitte wurde für 2 h bei RT geblockt in 1 x PBS mit 0,03 % Triton X-100 und 1 % Blocking Reagent (Roche), über Nacht mit Primärantikörpem in Blocking-Lösung (s.o.) bei 4 °C inkubiert, gewaschen, dann mit Speziesspezifischen Cy3, Alexa-488 oder FITC markierten Sekundärantikörpern in Blocking-Lösung für 2 h bei RT inkubiert, wieder gewaschen, mit 0,02 % Sudanschwarz zur Reduktion der Autofluoreszenz behandelt und mit Vectashield eingebettet. Die Analyse erfolgte am Fluoreszenz- (Nikon) bzw. Laser Scanning Mikroskop (Zeiss).

Folgende Primärantikörper wurden verwendet: Maus NF200 1:4000 (Sigma), Maus CGRP 1:1000 (Sigma), Kaninchen ATF-3 1:300 (SantaCruz). FITC-labeled *Griffonia simplicifolia* isolectin B4 (Sigma) 1:500, Kaninchen GFAP (1:1000, Chemicon), Kaninchen anti-Peripherin (1:1000, Chemicon), Kaninchen Iba-1 (1:500, DAKO).

Nach einer peripheren Nervenschädigung kommt es im ipsilateralen Rückenmark zu einer Mikrogliaaktivierung, die für die Entstehung neuropathischer Schmerzen bedeutsam ist. Diese aktivierten Mikrogliazellen zeigen eine starke Expression von Progranulin, gezeigt durch Microarray (Abb. 2), QRT-PCR (Abb. 2), Western Blot (Abb. 2) und in situ Hybridisierung (Abb. 3). Durch die Nervenläsion direkt geschädigte DRG- und Motoneuronen zeigen eine schwächere Expression (Abb. 3-4).

## Patentansprüche

1. Verwendung eines Granulins oder einer Granulin-ähnlichen Verbindung zur Herstellung einer pharmazeutischen Zusammensetzung zur Therapie oder Prophylaxe von neuropathischen Schmerzen,
wobei die Granulin-ähnliche Verbindung mindestens ein Molekül ist, das ausgewählt ist aus der Gruppe enthaltend Progranulin, Granulin 1, Granulin 2, Granulin 3, Granulin 4, Granulin 5, Granulin 6, Granulin 7, Paragranulin, Progranulinsignalmolekül, und zu den vorgenannten Moleküle gleichwirkenden Fragmenten dieser Moleküle.

2. Verwendung nach Anspruch 1 , wobei das Granulin oder die Granulin-ähnliche Verbindung eine für eines der genannten Moleküle kodierende Nukleinsäure ist.

3. Verwendung nach Anspruch 2, wobei die Nukleinsäure eine Nukleinsäure gemäß einer der SEQ. ID. NOs. 1 bis 10 oder eine zu einer dieser Sequenzen zu mindestens 50 % identische Nukleinsäure ist.

4. Verwendung nach Anspruch 1, wobei das Granulin oder die Granulin-ähnliche Verbindung ein Protein oder ein Peptid ist.

5. Verwendung nach Anspruch 4, wobei das Granulin oder die Granulin-ähnliche Verbindung ein Protein oder ein Peptid gemäß einer der SEQ. ID. NOs. 11 bis 20 oder eine zu einer dieser Sequenzen zu mindestens 50 % identisches Protein oder Peptid ist.

6. Verwendung nach einem der Ansprüche 1 bis 5 zur Herstellung von pharmazeutischen Zubereitungen zur Therapie oder Prophylaxe von neuropathischen Schmerzen bei Säugetieren, insbesondere bei Haus- und Nutztieren und beim Menschen.

7. Verwendung nach einem der Ansprüche 1 bis 6 zur Herstellung von pharmazeutischen Zubereitungen zur Reduktion neuropathischer Schmerzen.

8. Pharmazeutische Zusammensetzung, aufweisend ein Granulin oder ein gleichwirkendes Granulinfragment oder einen Granulin-Agonisten zur Verwendunginder Therapie oder Prophylaxe von neuropathischen Schmerzen bei Säugetieren, insbesondere bei Haus- und Nutztieren und beim Menschen,
wobei die Granulin-ähnliche Verbindung mindestens ein Molekül ist, das ausgewählt ist aus der Gruppe enthaltend Progranulin, Granulin 1, Granulin 2, Granulin 3, Granulin 4, Granulin 5, Granulin 6, Granulin 7, Paragranulin, Progranulinsignalmolekül, und zu den vorgenannten Moleküle gleichwirkenden Fragmenten dieser Moleküle.

9. Pharmazeutische Zusammensetzung zur Verwendung nach Anspruch 8, wobei das Granulin oder die Granulin-ähnliche Verbindung eine für eines der genannten Moleküle kodierende Nukleinsäure ist.

10. Pharmazeutische Zusammensetzung zur Verwendung nach Anspruch 9, wobei die Nukleinsäure eine Nukleinsäure gemäß einer der SEQ. ID. NOs. 1 bis 8 oder eine zu einer dieser Sequenzen zu mindestens 50 % identische Nukleinsäure ist.

11. Pharmazeutische Zusammensetzung zur Verwendung nach Anspruch 8, wobei das Granulin oder die Granulin-ähnliche Verbindung ein Protein oder ein Peptid ist.

12. Pharmazeutische Zusammensetzung zur Verwendung nach Anspruch 11, wobei das Granulin oder die Granulin-ähnliche Verbindung ein Protein oder ein Peptid gemäß einer der SEQ. ID. NOs. 11 bis 20 oder eine zu einer dieser Sequenzen zu mindestens 50 % identisches Protein oder Peptid ist.

13. Pharmazeutische Zusammensetzung zur Verwendung nach einem der Ansprüche 10 bis 12, in Form von Tabletten, Dragees, Pillen, Granulaten, Aerosolen, Infusionslösungen, Emulsionen, Suspensionen oder Lösungen.

## Claims

1. Use of a granulin or a granulin-like compound for producing a pharmaceutical composition for the therapy or prophylaxis of neuropathic pain, wherein said granulin-like compound is at least one molecule that is selected from the group containing progranulin, granulin 1, granulin 2, granulin 3, granulin 4, granulin 5, granulin 6, granulin 7, paragranulin, progranulin-signaling molecule, and fragments of these molecules that are functionally equivalent to the before-mentioned molecules.

2. The use according to claim 1, wherein said granulin or said granulin-like compound is a nucleic acid encoding for one of the said molecules.

3. The use according to claim 2, wherein the nucleic acid is a nucleic acid according to one of SEQ ID NOs 1 to 10 or a nucleic acid that is identical to at least 50% to one of these sequences.

4. The use according to claim 1, wherein said granulin or said granulin-like compound is a protein or a peptide.

5. The use according to claim 4, wherein said granulin or said granulin-like compound is a protein or a peptide according to one of SEQ ID NOs 11 to 20 or a protein or a peptide that is identical to at least 50% to one of these sequences.

6. Use according to any of claims 1 to 5 for producing pharmaceutical compositions for the therapy or prophylaxis of neuropathic pain in mammals, in particular in pets and livestock, and in the human.

7. The use according to any of claims 1 to 6 for producing pharmaceutical compositions for a reduction of neuropathic pain.

8. Pharmaceutical composition, comprising a granulin or a functionally equivalent granulin fragment or a granulin-agonist for use in the therapy or prophylaxis of neuropathic pain in mammals, in particular in pets and livestock, and in the human, wherein said granulin-like compound is at least one molecule selected from the group containing progranulin, granulin 1, granulin 2, granulin 3, granulin 4, granulin 5, granulin 6, granulin 7, paragranulin, progranulin signaling molecule, and fragments of these molecules that are functionally equivalent to these molecules.

9. The pharmaceutical composition for use according to claim 8, wherein said granulin or the granulin-like compound is a nucleic acid encoding for one of the said molecules.

10. The pharmaceutical composition for use according to claim 9, wherein said a nucleic acid is a nucleic acid according to one of SEQ ID NOs 1 to 8 or a nucleic acid that is identical to at least 50% to one of these sequences.

11. The pharmaceutical composition for use according to claim 8, wherein said granulin or said granulin-like compound is a protein or a peptide.

12. The pharmaceutical composition for use according to claim 11, wherein said granulin or said granulin-like compound is a protein or a peptide according to one of the SEQ ID NOs 11 to 20 or a protein or a peptide that is identical to at least 50% to one of these sequences.

13. The pharmaceutical composition for use according to any of claims 10 to 12, in the form of tablets, dragees, pills, granulates, aerosoles, infusion solutions, emulsions, suspensions or solutions.

## Revendications

1. Utilisation d'une granuline ou d'un composé analogue à la granuline pour la fabrication d'une composition pharmaceutique destinée à une thérapie ou à une prophylaxie de douleurs neuropathiques,
le composé analogue à la granuline étant au moins une molécule choisie dans le groupe constitué par la granuline 1, la granuline 2, la granuline 3, la granuline 4, la granuline 5, la granuline 6, la granuline 7, la paragranuline, la molécule signal de la progranuline, et les fragments de la molécule précité, agissant de manière identique à cette molécule.

2. Utilisation selon la revendication 1, la granuline ou le composé analogue à la granuline étant un acide nucléique codant pour l'une des molécules précitées.

3. Utilisation selon la revendication 2, l'acide nucléique étant un acide nucléique selon l'une des SEQ. ID. N °1 à 10 ou un acide nucléique identique à au moins 50 % à l'une de ces séquences.

4. Utilisation selon la revendication 1, la granuline ou le composé analogue à la granuline étant une protéine ou un peptide.

5. Utilisation selon la revendication 4, la granuline ou le composé analogue à la granuline étant une protéine ou un peptide selon l'une des SEQ. ID. N °11 à 20 ou une protéine ou un peptide étant identique à au moins 50 % à l'une de ces séquences.

6. Utilisation selon l'une des revendications 1 à 5 pour la fabrication de préparations pharmaceutiques destinées à la thérapie ou à la prophylaxie de douleurs neuropathiques chez des mammifères, notamment des animaux domestiques et de travail et chez l'homme.

7. Utilisation selon l'une des revendications 1 à 6 pour la fabrication de préparations pharmaceutiques destinées à la réduction de douleurs neuropathiques.

8. Composition pharmaceutique présentant une granuline, ou un fragment de granuline agissant de manière identique, ou un agoniste de granuline, pour une utilisation dans la thérapie ou dans la prophylaxie de douleurs neuropathiques chez des mammifères, en particulier, chez des animaux domestiques et de travail et chez l'homme,
le composé analogue à la granuline étant au moins une molécule choisie dans le groupe constitué par la granuline 1, la granuline 2, la granuline 3, la granuline 4, la granuline 5, la granuline 6, la granuline 7, la paragranuline, la molécule signal de la progranuline, et les fragments de la molécule précitée agissant de manière identique à cette molécule.

9. Composition pharmaceutique pour une utilisation selon la revendication 8, la granuline ou le composé analogue à la granuline étant un acide nucléique codant pour l'une des molécules précitées.

10. Composition pharmaceutique pour une utilisation selon la revendication 9, l'acide nucléique étant un acide nucléique selon l'une des SEQ. ID. N °1 à 8 ou un acide nucléique identique à au moins 50 % à l'une de ces séquences.

11. Composition pharmaceutique pour une utilisation selon la revendication 8, la granuline ou le composé analogue à la granuline étant une protéine ou un peptide.

12. Composition pharmaceutique pour une utilisation selon la revendication 11, la granuline ou le composé analogue à la granuline étant une protéine ou un peptide selon l'une des SEQ. ID. N °11 à 20 ou une protéine ou un peptide étant identique à au moins 50 % à l'une de ces séquences.

13. Composition pharmaceutique pour une utilisation selon l'une des revendications 10 à 12, sous la forme de comprimés, de dragées, de pilules, de granulés, d'aérosols, de solutions d'infusion, d'émulsions, de suspensions ou de solutions.


### SEQUENCE LISTING

<110> Johann Wolfgang Goethe-Universität Frankfurt am Main
<120> Verwendung eines Granulins oder einer Granulin-ähnlichen Verbindung zur Therapie oder Prophylaxe von chronischen Schmerzen
<130> FB22645
<150> DE 10 2007 033 359.7
   <151> 2007-07-16
<160> 20
<170> PatentIn version 3.3
<210> 1
   <211> 2323
   <212> DNA
   <213> Homo sapiens
<400> 1
   ggcgagagga agcagggagg agagtgattt gagtagaaaa gaaacacagc attccaggct      60
   ggccccacct ctatattgat aagtagccaa tgggagcggg tagccctgat ccctggccaa     120
   tggaaactga ggtaggcggg tcatcgcgct ggggtctgta gtctgagcgc tacccggttg     180
   ctgctgccca aggaccgcgg agtcggacgc aggcagacca tgtggaccct ggtgagctgg     240
   gtggccttaa cagcagggct ggtggctgga acgcggtgcc cagatggtca gttctgccct     300
   gtggcctgct gcctggaccc cggaggagcc agctacagct gctgccgtcc ccttctggac     360
   aaatggccca caacactgag caggcatctg ggtggcccct gccaggttga tgcccactgc     420
   tctgccggcc actcctgcat ctttaccgtc tcagggactt ccagttgctg ccccttccca    480
   gaggccgtgg catgcgggga tggccatcac tgctgcccac ggggcttcca ctgcagtgca     540
   gacgggcgat cctgcttcca aagatcaggt aacaactccg tgggtgccat ccagtgccct     600
   gatagtcagt tcgaatgccc ggacttctcc acgtgctgtg ttatggtcga tggctcctgg     660
   gggtgctgcc ccatgcccca ggcttcctgc tgtgaagaca gggtgcactg ctgtccgcac     720
   ggtgccttct gcgacctggt tcacacccgc tgcatcacac ccacgggcac ccaccccctg     780
   gcaaagaagc tccctgccca gaggactaac agggcagtgg ccttgtccag ctcggtcatg     840
   tgtccggacg cacggtcccg gtgccctgat ggttctacct gctgtgagct gcccagtggg     900
   aagtatggct gctgcccaat gcccaacgcc acctgctgct ccgatcacct gcactgctgc     960
   ccccaagaca ctgtgtgtga cctgatccag agtaagtgcc tctccaagga gaacgctacc    1020
   acggacctcc tcactaagct gcctgcgcac acagtggggg atgtgaaatg tgacatggag    1080
   gtgagctgcc cagatggcta tacctgctgc cgtctacagt cgggggcctg gggctgctgc     1140
   ccttttaccc aggctgtgtg ctgtgaggac cacatacact gctgtcccgc ggggtttacg    1200
   tgtgacacgc agaagggtac ctgtgaacag gggccccacc aggtgccctg gatggagaag     1260
   gccccagctc acctcagcct gccagaccca caagccttga agagagatgt cccctgtgat   1320
   aatgtcagca gctgtccctc ctccgatacc tgctgccaac tcacgtctgg ggagtggggc   1380
   tgctgtccaa tcccagaggc tgtctgctgc tcggaccacc agcactgctg cccccagggc   1440
   tacacgtgtg tagctgaggg gcagtgtcag cgaggaagcg agatcgtggc tggactggag   1500
   aagatgcctg cccgccgggc ttccttatcc caccccagag acatcggctg tgaccagcac   1560
   accagctgcc cggtggggca gacctgctgc ccgagcctgg gtgggagctg ggcctgctgc   1620
   cagttgcccc atgctgtgtg ctgcgaggat cgccagcact gctgcccggc tggctacacc   1680
   tgcaacgtga aggctcgatc ctgcgagaag gaagtggtct ctgcccagcc tgccaccttc   1740
   ctggcccgta gccctcacgt gggtgtgaag gacgtggagt gtggggaagg acacttctgc   1800
   catgataacc agacctgctg ccgagacaac cgacagggct gggcctgctg tccctaccgc   1860
   cagggcgtct gttgtgctga tcggcgccac tgctgtcctg ctggcttccg ctgcgcagcc   1920
   aggggtacca agtgtttgcg cagggaggcc ccgcgctggg acgccccttt gagggaccca   1980
   gccttgagac agctgctgtg agggacagta ctgaagactc tgcagccctc gggaccccac   2040
   tcggagggtg ccctctgctc aggcctccct agcacctccc cctaaccaaa ttctccctgg   2100
   accccattct gagctcccca tcaccatggg aggtggggcc tcaatctaag gccttccctg   2160
   tcagaagggg gttgtggcaa aagccacatt acaagctgcc atcccctccc cgtttcagtg   2220
   gaccctgtgg ccaggtgctt ttccctatcc acaggggtgt ttgtgtgtgt gcgcgtgtgc   2280
   gtttcaataa agtttgtaca ctttcaaaaa aaaaaaaaaa aaa                     2323
<210> 2
   <211> 165
   <212> DNA
   <213> Homo sapiens
<400> 2
   ggcccctgcc aggttgatgc ccactgctct gccggccact cctgcatctt taccgtctca     60
   gggacttcca gttgctgccc cttcccagag gccgtggcat gcggggatgg ccatcactgc    120
   tgcccacggg gcttccactg cagtgcagac gggcgatcct gcttc                    165
<210> 3
   <211> 171
   <212> DNA
   <213> Homo sapiens
<400> 3
   gccatccagt gccctgatag tcagttcgaa tgcccggact tctccacgtg ctgtgttatg 60
   gtcgatggct cctgggggtg ctgccccatg ccccaggctt cctgctgtga agacagggtg 120
   cactgctgtc cgcacggtgc cttctgcgac ctggttcaca cccgctgcat c 171
<210> 4
   <211> 168
   <212> DNA
   <213> Homo sapiens
<400> 4
   gtcatgtgtc cggacgcacg gtcccggtgc cctgatggtt ctacctgctg tgagctgccc 60
   agtgggaagt atggctgctg cccaatgccc aacgccacct gctgctccga tcacctgcac 120
   tgctgccccc aagacactgt gtgtgacctg atccagagta agtgcctc 168
<210> 5
   <211> 168
   <212> DNA
   <213> Homo sapiens
<400> 5
   gatgtgaaat gtgacatgga ggtgagctgc ccagatggct atacctgctg ccgtctacag 60
   tcgggggcct ggggctgctg cccttttacc caggctgtgt gctgtgagga ccacatacac 120
   tgctgtcccg cggggtttac gtgtgacacg cagaagggta cctgtgaa 168
<210> 6
   <211> 162
   <212> DNA
   <213> Homo sapiens
<400> 6
   gtcccctgtg ataatgtcag cagctgtccc tcctccgata cctgctgcca actcacgtct 60
   ggggagtggg gctgctgtcc aatcccagag gctgtctgct gctcggacca ccagcactgc 120
   tgcccccagg gctacacgtg tgtagctgag gggcagtgtc ag 162
<210> 7
   <211> 165
   <212> DNA
   <213> Homo sapiens
<400> 7
   atcggctgtg accagcacac cagctgcccg gtggggcaga cctgctgccc gagcctgggt 60
   gggagctggg cctgctgcca gttgccccat gctgtgtgct gcgaggatcg ccagcactgc 120
   tgcccggctg gctacacctg caacgtgaag gctcgatcct gcgag 165
<210> 8
   <211> 168
   <212> DNA
   <213> Homo sapiens
<400> 8
   gacgtggagt gtggggaagg acacttctgc catgataacc agacctgctg ccgagacaac     60
   cgacagggct gggcctgctg tccctaccgc cagggcgtct gttgtgctga tcggcgccac    120
   tgctgtcctg ctggcttccg ctgcgcagcc aggggtacca agtgtttg                 168
<210> 9
   <211> 90
   <212> DNA
   <213> Homo sapiens
<400> 9
   acgcggtgcc cagatggtca gttctgccct gtggcctgct gcctggaccc cggaggagcc     60
   agctacagct gctgccgtcc ccttctggac                                      90
<210> 10
   <211> 51
   <212> DNA
   <213> Homo sapiens
<400> 10
   atgtggaccc tggtgagctg ggtggcctta acagcagggc tggtggctgg a 51
<210> 11
   <211> 593
   <212> PRT
   <213> Homo sapiens
<400> 11
<210> 12
   <211> 56
   <212> PRT
   <213> Homo sapiens
<400> 12
   Gly Gly Pro Cys Gln Val Asp Ala His Cys Ser Ala Gly His Ser Cys
   1               5                   10                  15
   Ile Phe Thr Val Ser Gly Thr Ser Ser Cys Cys Pro Phe Pro Glu Ala
               20                  25                  30
   Val Ala Cys Gly Asp Gly His His Cys Cys Pro Arg Gly Phe His Cys
           35                  40                  45
   Ser Ala Asp Gly Arg Ser Cys Phe
       50                  55
<210> 13
   <211> 57
   <212> PRT
   <213> Homo sapiens
<400> 13
   Ala Ile Gln Cys Pro Asp Ser Gln Phe Glu Cys Pro Asp Phe Ser Thr
   1               5                   10                  15
   Cys Cys Val Met Val Asp Gly Ser Trp Gly Cys Cys Pro Met Pro Gln
               20                  25                  30
   Ala Ser Cys Cys Glu Asp Arg Val His Cys Cys Pro His Gly Ala Phe
           35                  40                  45
   Cys Asp Leu Val His Thr Arg Cys Ile
       50                  55
<210> 14
   <211> 56
   <212> PRT
   <213> Homo sapiens
<400> 14
   Val Met Cys Pro Asp Ala Arg Ser Arg Cys Pro Asp Gly Ser Thr Cys
   1               5                   10                  15
   Cys Glu Leu Pro Ser Gly Lys Tyr Gly Cys Cys Pro Met Pro Asn Ala
               20                  25                  30
   Thr Cys Cys Ser Asp His Leu His Cys Cys Pro Gln Asp Thr Val Cys
           35                  40                  45
   Asp Leu Ile Gln Ser Lys Cys Leu
       50                  55
<210> 15
   <211> 56
   <212> PRT
   <213> Homo sapiens
<400> 15
   Asp Val Lys Cys Asp Met Glu Val Ser Cys Pro Asp Gly Tyr Thr Cys
   1               5                   10                  15
   Cys Arg Leu Gln Ser Gly Ala Trp Gly Cys Cys Pro Phe Thr Gln Ala
               20                  25                  30
   Val Cys Cys Glu Asp His Ile His Cys Cys Pro Ala Gly Phe Thr Cys
           35                  40                  45
   Asp Thr Gln Lys Gly Thr Cys Glu
       50                  55
<210> 16
   <211> 54
   <212> PRT
   <213> Homo sapiens
<400> 16
   Val Pro Cys Asp Asn Val Ser Ser Cys Pro Ser Ser Asp Thr Cys Cys
   1               5                   10                  15
   Gln Leu Thr Ser Gly Glu Trp Gly Cys Cys Pro Ile Pro Glu Ala Val
               20                  25                  30
   Cys Cys Ser Asp His Gln His Cys Cys Pro Gln Gly Tyr Thr Cys Val
           35                  40                  45
   Ala Glu Gly Gln Cys Gln
       50
<210> 17
   <211> 55
   <212> PRT
   <213> Homo sapiens
<400> 17
   Ile Gly Cys Asp Gln His Thr Ser Cys Pro Val Gly Gln Thr Cys Cys
   1               5                   10                  15
   Pro Ser Leu Gly Gly Ser Trp Ala Cys Cys Gln Leu Pro His Ala Val
               20                  25                  30
   Cys Cys Glu Asp Arg Gln His Cys Cys Pro Ala Gly Tyr Thr Cys Asn
           35                  40                  45
   Val Lys Ala Arg Ser Cys Glu
       50                  55
<210> 18
   <211> 56
   <212> PRT
   <213> Homo sapiens
<400> 18
   Asp Val Glu Cys Gly Glu Gly His Phe Cys His Asp Asn Gln Thr Cys
   1               5                   10                  15
   Cys Arg Asp Asn Arg Gln Gly Trp Ala Cys Cys Pro Tyr Arg Gln Gly
               20                  25                  30
   Val Cys Cys Ala Asp Arg Arg His Cys Cys Pro Ala Gly Phe Arg Cys
           35                  40                  45
   Ala Ala Arg Gly Thr Lys Cys Leu
       50                  55
<210> 19
   <211> 30
   <212> PRT
   <213> Homo sapiens
<400> 19
   Thr Arg Cys Pro Asp Gly Gln Phe Cys Pro Val Ala Cys Cys Leu Asp
   1               5                   10                  15
   Pro Gly Gly Ala Ser Tyr Ser Cys Cys Arg Pro Leu Leu Asp
               20                  25                  30
<210> 20
   <211> 17
   <212> PRT
   <213> Homo sapiens
<400> 20
   Met Trp Thr Leu Val Ser Trp Val Ala Leu Thr Ala Gly Leu Val Ala
   1               5                   10                  15
   Gly